# FASCICULE DE BREVET EUROPEEN

(11) **EP 4 061 398 B1**
(45) Date de publication et mention de la délivrance du brevet: **31.07.2024**
(21) Numéro de dépôt: 20806920.3
(22) Date de dépôt: 19.11.2020
(51) Int. Cl.: A61K 31/555, A61K 33/243, A61K 38/16, A61K 35/748, A61P 1/16, A61P 9/00, A61P 13/12, A61P 25/02, A61P 39/06

(54) **EXTRAIT LIQUIDE AQUEUX DE SPIRULINE POUR LA PRÉVENTION ET/OU LE TRAITEMENT DES NEUROPATHIES PÉRIPHÉRIQUES CHIMIO-INDUITES ET LEURS SYMPTÔMES, COMPOSITION ET UTILISATION CORRESPONDANTES**
WÄSSRIGER FLÜSSIGEXTRAKT VON SPIRULINA ZUR VORBEUGUNG UND/ODER BEHANDLUNG PERIPHERER CHEMOTHERAPIEINDUZIERTER NEUROPATHIEN UND DEREN SYMPTOMEN SOWIE ENTSPRECHENDE ZUSAMMENSETZUNG UND VERWENDUNG
AQUEOUS LIQUID EXTRACT OF SPIRULINA FOR PREVENTING AND/OR TREATING PERIPHERAL CHEMOTHERAPY-INDUCED NEUROPATHIES AND THE SYMPTOMS THEREOF, AND CORRESPONDING COMPOSITION AND USE

(30) Priorité: 19.11.2019 FR 1912891
(43) Date de publication de la demande: 28.09.2022
(73) Titulaire: Algosource, 44602 Saint-Nazaire (FR)
(72) Inventeur: LÉPINE, Olivier, 44600 SAINT-NAZAIRE (FR)
(74) Mandataire: Vidon Brevets & Stratégie
(86) Numéro de dépôt international: PCT/EP2020/082661
(87) Numéro de publication internationale: WO 2021/099453

(56) Documents cités:
- WO-A1-2015/140265
- FR-A1- 2 957 077
- FERNANDEZ-ROJAS B. ET AL.: "C-Phycocyanine prevents cisplatine-induced nephrotoxicity through inhibition of oxidative stress.", FOOD FUNCTIO, vol. 5, 2014, pages 480 - 490, XP035513115

## Description

### Domaine technique de l'invention

Le domaine de l'invention est celui du traitement et/ou de la prévention des effets secondaires indésirables induits par un traitement thérapeutique anti-cancéreux, et en particulier un traitement de type chimiothérapeutique.

Plus particulièrement, l'invention concerne le traitement et/ou la prévention des effets secondaires indésirables induits par l'administration d'un composé anti-cancéreux, et en particulier la prévention et/ou le traitement de(s) neuropathie(s) périphérique(s) induite(s) par les chimiothérapies, ou neuropathie(s) chimio-induite(s).

Plus précisément, l'invention concerne l'utilisation d'un extrait liquide aqueux obtenu à partir de cyanobactéries, ou Spiruline, renfermant de la Phycocyanine et optionnellement des polysaccharides de Spiruline, pour la prévention et/ou le traitement de(s) neuropathie(s) périphérique(s) induite(s) par les chimiothérapies; une composition comprenant ledit extrait liquide aqueux pour utilisation dans la prévention et/ou le traitement des effets secondaires indésirables induits par un traitement thérapeutique anti-cancéreux, et en particulier les neuropathies périphériques induites par les chimiothérapies.

### Art antérieur

La chimiothérapie représente actuellement la modalité la plus souvent utilisée dans le traitement du cancer. Elle peut être utilisée seule ou en association avec d'autres approches thérapeutiques (chirurgie, radiothérapie, thérapie ciblée...).

La chimiothérapie est un traitement comportant l'administration de médicaments qui agissent sur les cellules cancéreuses, soit en les détruisant, soit en les empêchant de se multiplier. Cependant, ces médicaments agissent également sur les autres cellules de l'organisme qui se développent rapidement, et cela explique les effets secondaires indésirables de la chimiothérapie.

En effet, les effets secondaires sont liés à l'action des médicaments anti-cancéreux sur les cellules qui se multiplient rapidement, c'est-à-dire les cellules cancéreuses mais également celles de la moelle osseuse, des cheveux, de la peau, etc. Les effets secondaires de la chimiothérapie sont fréquents et d'intensité variable selon les médicaments utilisés, les dosages, les modes d'administration et les personnes.

Par « effet secondaire », on entend un effet indésirable d'un médicament ou d'un soin qui induit ou potentiellement induit un effet défavorable par le traitement (par exemple gêne, allergie, complications graves, y compris le décès). Cet effet secondaire peut être immédiat ou différé.

Les effets secondaires les plus fréquents consistent en des modifications de la formule sanguine (diminution du taux de certains globules blancs, des globules rouges et des plaquettes), des troubles digestifs (nausées, vomissements, diarrhée, constipation), chute des cheveux, fatigue, sensations d'engourdissement ou de fourmillement. D'autres effets secondaires sont possibles tels que des troubles au niveau des reins, du coeur ou du foie. Par exemple, il est connu que les sels de platine (cisplatine, carboplatine et oxaliplatine) et les taxanes (paclitaxel et docétaxel) sont neurotoxiques et à l'origine du développement de neuropathies chez les patients. Les anthracyclines (l'épirubicine, la pirarubicine, l'idarubicine, la zorubicine, l'aclarubicine, la doxorubicine) sont quant à elles connues pour favoriser une peroxydation lipidique et être cardio-toxiques.

Les neuropathies dites « chimio-induites » sont parmi les effets secondaires les plus sévères. On entend par « neuropathies chimio-induites » les neuropathies provoquées par l'administration ou l'exposition à un composé neurotoxique, ce composé pouvant être :
- soit un composé médicamenteux, en particulier un composé anti-cancéreux présentant une neurotoxicité ;
- soit un composé non médicamenteux tels que l'alcool, les organophosphorés, les métaux lourds (Pb, As, Hg, Tl), les toxines fongiques ou bactériennes, certains solvants comme le toluène, etc.

Ce type de neuropathies se traduit par une atteinte principalement sensitive, une persistance des paresthésies et des sensations d'engourdissement entre les cures de chimiothérapie, ainsi que des douleurs spontanées. Dans les cas les plus sévères, les patients développent une ataxie sensorielle accompagnée d'une perte de la sensibilité superficielle et profonde affectant le moindre geste du quotidien.

La douleur neuropathique, comme l'allodynie, l'hyperesthésie, la dysesthésie et l'ataxie, est un symptôme de la neuropathie, défini comme une douleur secondaire à une lésion ou un état pathologique affectant le système somatosensoriel. Elle est secondaire à des lésions périphériques (fibres nerveuses afférentes) ou centrales (centres nerveux impliqués dans la transmission et/ou la régulation du message douloureux, par exemple au niveau du thalamus).

Les étiologies de la douleur neuropathique sont diverses. Elles peuvent être causées par une lésion physique comme c'est le cas lors d'un accident, un acte chirurgical, une amputation (membre fantôme). Elles peuvent également être chimio-induites comme c'est le cas suite à un abus d'alcool, une intoxication aux métaux (arsenic ou thallium par exemple), l'exposition à un agent chimique environnemental (organophosphoré par exemple), un médicament, une toxine fongique ou bactérienne. Elles peuvent également faire suite à un état pathologique acquis ou héréditaire (par exemple une amylose, le syndrome de Guillain-Barré, une infection au VIH, une infection bactérienne, un zona, un herpès, un diabète, une maladie auto-immune et/ou une maladie de Fabry).

La demande internationale WO 2015/140265 concerne une composition pour utilisation dans le traitement et/ou la prévention des neuropathies chimio-induites. En particulier, cette composition comprend un inhibiteur réversible de l'acétylcholinestérase d'action centrale.

En ce qui concerne les neuropathies (notamment les douleurs neuropathiques et troubles de la sensibilité associés) induites par les chimiothérapies telles celles aux sels de platine, il semblerait qu'elles soient liées, de manière directement dépendante, à la durée de la cure et à la dose administrée.

Afin de limiter ces effets secondaires indésirables on procède généralement à une adaptation posologique, c'est-à-dire à une diminution des doses administrées, un espacement des cures, voire l'interruption des cures. Or cette adaptation posologique compromet les chances de rémission et de survie du patient. C'est le cas par exemple pour limiter, voire supprimer, les symptômes des neuropathies induites par les traitements aux sels de platine.

La Phycocyanine, ou C-Phycocyanine, est une protéine de la famille des phycobiliprotéines caractérisée par son activité antioxydante (« C-Phycocyanine prevents cisplatine-induced nephrotoxicity through inhibition of oxidative stress. » Fernandez-Rojas B. et al, Food Functio 5 :480-490 (2014)) et par une couleur bleue intense (due à un pic d'absorption caractéristique à 620 nm). La Phycocyanine est un des principaux pigments d'une micro-algue procaryote, ou cyanobactérie, communément appelée Spiruline, utilisée comme complément alimentaire en raison de sa haute teneur en protéines.

En effet, la Phycocyanine est utilisée dans l'industrie alimentaire, les cosmétiques, la biotechnologie et la pratique médicale. Certaines de ses propriétés nutraceutiques sont, entre autres, anti-oxydantes, anti-inflammatoires, anti-néoplasiques et anti-diabétiques. En effet, son activité anti-oxydante est impliquée dans l'effet protecteur contre les lésions hépatiques, neuronales et rénales. En particulier, il a été démontré que la Phycocyanine possède plusieurs propriétés nutraceutiques telles que renoprotectrices (contre l'oxalate et le cisplatine) (« Salubrious effect of C-phycocyanine against oxalate-mediated rénal cell injury. » Farooq S et al, Clin Chim Acta 348 :199-205 (2004); « C-Phycocyanine atténuâtes cisplatin-induced nephroroxicity in mice. » Lim BJ, et al, Ren Fail 34 :892-900 (2012)), anti-inflammatoires (« Furtherstudies on anti-inflammatory activity of phycocyanine in some animal models of inflammation. » Romay C et al, Inflamm Res 47 :334-338 (1998)) et hépatoprotectrices (contre le tétrachlorure de carbone) *(*« Protective effect of C-Phycocyanine against carbon tetrachloride-induced hepatocyte damage in vitro and in vivo » Ou Y, et al, Chem Biol Interact 185 :94-100, (2010)).

Il a également été démontré que la Phycocyanine joue un rôle protecteur contre certains effets secondaires liés à des composés anti-cancéreux comme par exemple le cisplatine. En particulier il a été démontré un rôle protecteur de la Phycocyanine contre la dysfonction mitochondriale rénale induite par le cisplatine, lorsqu'elle est administrée à une dose de 22-30mg/kg de masse corporel (« C-Phycocyanine prevents cisplatin-induced mitochondrial dysfunction and oxidative stress », Fernandez-Rojas B. et al, Mol Cell Biochem (2015)).

Il a enfin été démontré que la Phycocyanine, lorsqu'elle est administrée à une dose de 5-30mg/kg de masse corporel, joue un rôle dans la prévention du stress oxydatif et la diminution de l'activité des enzymes anti-oxydantes dans la néphrotoxicité induite par le cisplatine.

Ainsi, ces données suggèrent que la consommation humaine de Phycocyanine peut être utile pour la prévention et/ou le traitement des maladies rénales associées au stress oxydatif induit par un traitement chimiothérapeutique impliquant le cisplatine. (« C-Phycocyanin prevents cisplatin-induced nephrotoxicity through inhibition of oxidative stress. Berenice Fernandez-Rojas et al, Food Funct, 2014 »).

Jusqu'à présent, les traitements pour réduire les effets secondaires liés à la chimiothérapie visaient les effets secondaires de manière individuelle (par exemple nausée, vomissement, perte de cheveux...) et ne parvenaient pas nécessairement à agir plus en amont pour traiter leur cause pouvant être commune (comme par exemple induction d'un stress oxydatif provoquant la mort cellulaire de cellules saines) de manière efficace et ciblée.

La demande de brevet français FR2957077 concerne l'administration d'un composé, le riluzole, dans le traitement et/ou la prévention des effets secondaires de chimiothérapies basée sur des agents anti-cancéreux neurotoxique.

La Phycocyanine, grâce à son activité anti-oxydante, permet de diminuer certains effets secondaires liés à des composés anti-cancéreux comme par exemple le cisplatine. Cependant, un effet protecteur de la Phycocyanine n'est observé qu'à des dosages très élevés compris entre 350-900 mg pour un adulte d'environ 70 kg, ce qui est contraignant en termes de coût et de production à l'échelle industrielle, mais a également des conséquences sur le traitement du patient (par exemple posologie élevée).

### Objectifs de l'invention

L'invention a notamment pour objectif de pallier ces inconvénients de l'art antérieur et de proposer une alternative thérapeutique à la simple adaptation posologique.

Plus précisément, l'invention a pour objectif, dans au moins un mode de réalisation, de fournir une composition permettant de prévenir et/ou d'éliminer, ou à tout le moins de réduire davantage, les effets secondaires indésirables induits par un composé anti-cancéreux.

En particulier, l'invention a pour objectif, dans au moins un mode de réalisation, de fournir une composition permettant de prévenir et/ou d'éliminer, ou à tout le moins de réduire, les neuropathies périphériques induite par l'administration d'un composé anti-cancéreux, et en particulier un composé anti-cancéreux choisi parmi les sels de platine.

Un autre objectif de l'invention, dans au moins un mode de réalisation, est de fournir une composition permettant de prévenir et/ou d'éliminer, ou à tout le moins de réduire davantage, les effets secondaires indésirables induits par un composé anti-cancéreux avec un dosage en Phycocyanine plus faible que l'art antérieur.

### Présentation de l'invention

Ces objectifs, ainsi que d'autres qui apparaîtront par la suite, sont atteints à l'aide d'une composition pour utilisation dans le traitement et/ou la prévention des neuropathies périphériques chimio-induites et leurs symptômes induits par un composé anti-cancéreux, ladite composition comprenant un extrait liquide aqueux, ledit extrait liquide aqueux comprenant de la Phycocyanine.

En effet, la Phycocyanine, grâce notamment à ses effets antioxydants, permet de diminuer les effets secondaires indésirables induits par un composé anti-cancéreux.

Selon une première caractéristique particulière, l'extrait aqueux comprend en outre des polysaccharides de Spiruline.

De manière avantageuse les polysaccharides de Spiruline potentialisent l'effet de la Phycocyanine rendant l'extrait liquide aqueux selon l'invention particulièrement efficace dans le traitement et/ou la prévention des effets secondaires induits par un composé anti-cancéreux et en particulier des neuropathies périphériques chimio-induites et de leurs symptômes.

En effet, les inventeurs ont mis en avant de manière inattendue qu'une telle composition comprenant un extrait liquide aqueux de cyanobactéries, également connu sous la dénomination Spirulysat^{®} et commercialisé par la société AlgoSource, lorsqu'il est pris en complément d'un traitement thérapeutique anti-cancéreux, tel que notamment un traitement chimiothérapeutique, permet de réduire encore plus efficacement les effets secondaires indésirables de ce traitement chimiothérapeutique.

Par la suite, on désigne par le terme Spirulysat^{®} l'extrait liquide aqueux selon l'invention, i.e comprenant de la Phycocyanine et optionnellement des polysaccharides de Spiruline.

Cette diminution sensible des effets secondaires indésirables liés aux traitements anti-cancéreux a notamment comme conséquence positive la possibilité de soumettre les patients sous traitement anti-cancéreux, comme par exemple une chimiothérapie, combinée ou non avec une radiothérapie, à l'ensemble des cycles nécessaires au traitement.

En outre, les patients dont l'état de santé ne permet plus de recevoir de traitements lourds (chimiothérapie combinée ou non avec une radiothérapie) ont vu, grâce au Spirulysat^{®}, leur état se stabiliser. Ainsi, les inventeurs ont mis en avant que l'administration de Spirulysat^{®}, sans constituer un traitement curatif, offrait une chance de survie supplémentaire significative offerte aux malades.

Par ailleurs, le Spirulysat^{®} est très bien accepté par tous les patients même en cas de troubles digestifs sévères (nausées, vomissements, douleurs abdominales, diarrhées, ...).

Les inventeurs ont mis en avant que le rôle du Spirulysat^{®} dans la diminution des effets secondaires indésirables liés à des traitement thérapeutiques anti-cancéreux, comme par exemple les chimiothérapies, serait dû notamment à ses fonctionnalités antioxydantes puissantes liées à la présence de Phycocyanine. En effet, les fonctionnalités antioxydantes du Spirulysat^{®} limitent le stress oxydatif ce qui permet notamment de protéger les organes lors de chimiothérapie.

Les fonctionnalités antioxydantes du Spirulysat^{®} ont notamment été démontrées à travers l'étude « Spirox » menée par les sociétés AlgoSource et Biofortis Mérieux NutriSciences (« Study to Assess Antioxidant Efficacy of Spirulina on oxLDL and Lipids Metabolism on Subjects With Metabolic Syndrome (SPIROX) », 2016). Cette étude clinique a été réalisée en France sur 40 personnes pour démontrer l'activité antioxydante du Spirulysat^{®}. En particulier, cette étude a montré l'effet du Spirulysat^{®} sur le stress oxydatif et le métabolisme lipidique chez des sujets présentant un syndrome métabolique. Cette étude a été réalisée en double aveugle contre placébo, avec un résultat concluant sur le marqueur des isoprostanes urinaires (marqueur de l'oxydation lipidique). Cette étude démontre l'activité antioxydante du Spirulysat^{®} pour deux ampoules par jour (pendant 12 semaines) soit : 20 mg de Phycocyanine par jour et 3 mg de polysaccharides de Spiruline par jour.

En effet, la prise de Spirulysat^{®} en complément d'un traitement anti-cancéreux, comme par exemple chimiothérapeutique, stimule le système immunitaire, protège les organes tels que les reins, le foie, les nerfs ou les neurones, ce qui a pour effet de diminuer l'apparition d'effets secondaires indésirables. En particulier, les inventeurs ont démontré un rôle du Spirulysat^{®} sur la diminution des neuropathies périphériques chimio-induites et leurs symptômes.

Ces effets secondaires indésirables appartiennent au groupe comprenant les troubles du système immunitaire, les troubles des reins, du foie, du coeur et les neuropathies périphériques chimio-induites et leurs symptômes, ou leur combinaison.

Dans un exemple, l'action du Spirulysat^{®} sur le foie a été démontrée notamment par une étude menée au centre de Recherche en nutrition humaine grand ouest et publiées dans la revue Nutrients (Marni Coué et al, « Spirulina Liquid Extract Protects against Fibrosis Related to Non-Alcoholic Steatohepatitis and Increases Ursodeoxycholic Acid », Nutirents 2018). Les troubles sur le foie due à un stress oxydatif de nature chimique ou par un régime hypercalorique ont été prévenus par le Spirulysat^{®}.

En particulier, selon une caractéristique de l'invention, les effets secondaires indésirables sont choisis parmi les neuropathies périphériques chimio-induites et leurs symptômes.

Selon une autre caractéristique, le composé anti-cancéreux appartient au groupe comprenant un sel de platine, un taxane, une anthracycline.

Avantageusement, le Spirulysat^{®} permet de traiter et/ou prévenir des effets secondaires indésirables liés aux chimiothérapies. En particulier, des bienfaits de la composition selon l'invention ont été observés sur le fonctionnement des reins et du cerveau lors de chimiothérapies faisant intervenir les sels de platine, comme par exemple cisplatine, oxaliplatine et carboplatine.

De même, un effet protecteur a été observé sur le cerveau, principalement lors d'un traitement oncologique à base de taxanes, comme par exemple le paclitaxel et le docétaxel.

Un effet protecteur sur le coeur a également été observé lors de traitement oncologique à base d'anthracyclines, comme par exemple l'épirubicine.

Plus particulièrement, le composé anti-cancéreux est choisi parmi les sels de platine ; de préférence parmi le groupe comprenant le cisplatine, la carboplatine, l'oxaliplatine et leur mélange.

Avantageusement, la composition selon l'invention permet de réduire les effets secondaires indésirables des chimiothérapies à base de sels de platine, et plus spécifiquement, les chimiothérapies faisant intervenir l'oxaliplatine.

Selon une autre caractéristique de l'invention, l'extrait aqueux liquide comprend entre 20 et 150 mg, préférentiellement entre 50 et 120 mg, très préférentiellement 100 mg de Phycocyanine.

Préférentiellement, l'extrait aqueux liquide comprend en outre entre 5 et 25 mg, très préférentiellement 20 mg de polysaccharides de Spiruline.

Selon une autre caractéristique la composition comprend uniquement ledit extrait aqueux liquide et elle est préférentiellement administrée sous forme liquide par voie orale.

Avantageusement, la composition selon l'invention est administrée par voie orale, ce qui lui permet d'être bien supportée par le patient et d'être simple à administrer. De plus, cette composition est prise comme un complément alimentaire ne nécessitant pas d'assistance médicale.

Préférentiellement, la composition comprenant uniquement ledit extrait aqueux liquide est administrée à une dose comprise entre 20 mg/jour et 150mg/jour, préférentiellement entre 50mg/jour et 150mg/jours, très préférentiellement à 100mg/jour.

Ainsi, la composition selon l'invention permet d'obtenir des effets sur la diminution des effets secondaires induits par des composés anti-cancéreux, et notamment les neuropathies périphériques induites par les sels de platine, avec des doses de Phycocyanine au moins 6 à 18 fois inférieure à celles décrient dans l'art antérieur.

Avantageusement, plusieurs niveaux de concentrations de Spirulysat^{®} ont été testés, et une relation effet/dose a été observée.

En particulier, les inventeurs ont mis en évidence une action très significative du Spirulysat^{®} concentré à une dose de 2 à 5 fois supérieure aux valeurs de 20mg/jour et 50mg/jour, dans la diminution des nombreux effets secondaires des traitements chimiothérapeutiques.

L'invention concerne également un extrait liquide aqueux tel que défini selon la revendication 10 pour son utilisation telle que définie dans la même revendication.

Préférentiellement, le composé anti-cancéreux appartient au groupe comprenant un sel de platine, un taxane, une anthracycline, en particulier le composé anti-cancéreux est choisi parmi les sels de platine ; de préférence parmi le groupe comprenant le cisplatine, la carboplatine, l'oxaliplatine et leur mélange.

### Description détaillée de modes de réalisation

Le principe général de l'invention repose sur l'administration d'une composition comprenant un extrait liquide aqueux obtenu à partir de cyanobactéries, cet extrait aqueux comprenant de la Phycocyanine et optionnellement des polysaccharides de Spiruline, pour utilisation dans le traitement et/ou la prévention des effets secondaires indésirables induits par un composé anti-cancéreux, en particulier pour prévenir et/ou traiter les neuropathies périphériques chimio-induites et leurs symptômes associés, comme les douleurs neuropathiques.

L'invention concerne plus particulièrement une composition comprenant uniquement l'extrait aqueux pour utilisation dans le traitement et/ou la prévention des neuropathies périphériques induites par un traitement aux sels de platine comme par exemple l'oxaliplatine.

En particulier, cet extrait liquide aqueux de cyanobactéries, ou Spiruline, comprend de la Phycocyanine et très préférentiellement des polysaccharides de Spiruline, et il est commercialisé par la Société AlgoSource sous la dénomination Spirulysat^{®}. Le Spirulysat^{®} est un extrait aqueux liquide, dont le procédé d'extraction est décrit dans la demande de brevet FR3064269. Ce procédé d'extraction particulier permet d'extraire la Phycocyanine sans la dénaturer, c'est-à-dire en conservant sa structure spatiale. Ainsi le Spirulysat^{®} est particulièrement riche en Phycocyanine de meilleure qualité, ce qui lui confère une efficacité accrue.

Cet extrait aqueux liquide est obtenu à partir de cyanobactéries, ou micro-algues. En particulier, le Spirulysat^{®} est obtenu à partir de cyanobactéries choisies parmi la spiruline Arthrospira platensis, l'Aphanizomenon flos-aquae et le Phormidium molle. Les cyanobactéries sont des micro-algues procaryotes également appelées « algues bleues ». Le terme « Spiruline » peut désigner plusieurs espèces différentes de cyanobactéries filamenteuses, comme par exemple les espèces de deux genres distincts : Arthrospira et Spirulina. Par la suite, on désigne par « Spiruline » les cyanobactéries utilisées pour l'obtention du Spirulysat^{®}. La Spiruline est connue pour être très riche en molécules anti-oxydantes comme la Phycocyanine, une phycobiliprotéine d'un bleu intense.

Par « polysaccharides » (parfois appelés glycanes, polyosides, polyholosides ou glucides complexes), on entend des polymères de la famille des glucides constitués de plusieurs oses liés entre eux par des liaisons osidiques.

Dans un mode de réalisation selon l'invention, l'extrait aqueux liquide comprend entre 20 et 150 mg, préférentiellement entre 50 et 120 mg, très préférentiellement 100 mg de Phycocyanine. Préférentiellement, l'extrait aqueux liquide comprend en outre entre 5 et 25 mg, très préférentiellement 20 mg de polysaccharides de Spiruline.

Le Spirulysat^{®} est administrée préférentiellement sous forme liquide par voie orale. En particulier, la composition est administrée à une dose comprise entre 20 mg/jour et 150mg/jour, préférentiellement entre 50mg/jour et 150mg/jours, très préférentiellement à 100mg/jour.

En particulier, l'invention comprend, dans un de ses aspects particulièrement intéressants, l'administration de la composition comprenant le Spirulysat^{®} en amont et/ou en parallèle de l'administration d'un composé anti-cancéreux, en particulier un composé anti-cancéreux choisi parmi les sels de platine, les taxanes, les anthracyclines ou leur mélange.

Les sels de platine tels que l'oxaliplatine, le carboplatine et le cisplatine sont actuellement utilisés pour le traitement de certaines formes de cancer. Ils présentent tous une neurotoxicité plus ou moins importante en fonction des molécules, l'oxaliplatine étant le plus neurotoxique des sels de platine. Cette neurotoxicité se traduit par l'apparition de signes rencontrés dans les douleurs neuropathiques comme des sensations de brûlures, de fourmillements, un engourdissement, l'allodynie, la paresthésie, qui persistent bien après l'arrêt du traitement. Cette neurotoxicité se manifeste de manière aigüe suite à chaque administration d'oxaliplatine au patient, et devient chronique au fur et à mesure que le patient reçoit des doses de sels de platines (cisplatine, carboplatine et oxaliplatine).

Dans un mode de réalisation préféré, le Spirulysat^{®} est administré avant l'administration du composé anti-cancéreux, en particulier avant l'administration de sels de platine, comme l'oxaliplatine. Cette option permet de potentialiser les effets du Spirulysat^{®} en lui laissant le temps d'agir et en évitant que la neuropathie périphérique et les symptômes associés ne se développent. L'administration prophylactique du Spirulysat^{®}, avant l'administration d'au moins un composé anti-cancéreux, notamment un sel de platine comme l'oxaliplatine, permet d'éviter le développement des neuropathies périphériques et des symptômes associés, comme par exemple les douleurs neuropathiques.

Avantageusement, le Spirulysat^{®} n'est pas administré le jour de l'administration du composé anti-cancéreux, notamment un sel de platine, afin de laisser le traitement anti-cancéreux agir, ni le jour suivant cette administration.

L'administration du Spirulysat^{®} reprend deux ou trois jours après l'administration du composé anti-cancéreux, notamment un sel de platine, et ce tous les jours jusqu'à l'administration suivante du composé anti-cancéreux, notamment un sel de platine.

Les inventeurs ont observé que l'administration d'une dose de Spirulysat^{®} avant le début du traitement anti-cancéreux comprenant l'injection de sels de platine, puis quotidiennement entre les injections de sels de platine permettait de réduire l'apparition de neuropathies périphériques causées par ces sels de platine, et d'empêcher le développement de neuropathies et donc d'une douleur chronique.

Dans un exemple d'administration du Spirulysat^{®}, celui-ci est administré sous la forme par exemple d'ampoule comprenant 10 ml de l'extrait liquide aqueux soit environ 10mg de Phycocyanine et 1,5mg de polysaccharides de Spiruline. L'invention n'étant pas limitée à ce mode d'administration, ni à la composition et/ou au volume de l'ampoule.

Dans un autre exemple d'administration du Spirulysat^{®}, celui-ci peut être administré sous la forme d'ampoule de 5 ml de l'extrait liquide aqueux.

Dans d'autres exemples d'administration, le Spirulysat^{®} peut être administré dans un flacon de verre, sous forme de gélule contenant l'extrait liquide aqueux, ou encore de boisson contenant l'extrait liquide aqueux.

En effet, avec une prise de 2 ampoules/jour, soit environ 20mg/jour de Phycocyanine et 3 mg/jours de polysaccharide de Spiruline, des effets ont été observés, mais de façon limitée chez certains patients.

Sur l'Homme, pendant un traitement chimiothérapeutique, notamment un traitement aux sels de platine et plus particulièrement à l'oxaliplatine, une dose de 5 à 6 ampoules par jour, c'est-à-dire 50mg-60mg/jour de Phycocyanine et 7,5mg/jours-9mg/jour de polysaccharides de Spiruline, donne des résultats bien meilleurs, qu'une dose de 2 ampoules par jours, soit 20mg/jour de Phycocyanine et 3 mg/jours de polysaccharides de Spiruline.

Sur les animaux de compagnie comme par exemple les chats, chiens, hamsters, NAC (nouveaux animaux de compagnie, une dose de 0.1 ml de Spirulysat^{®} par kg de masse corporelle a été identifiée comme active. Ce qui correspondant aux mêmes ordres de grandeur : 7 ml pour 70 kg. Des doses équivalentes à celle administrées chez l'Homme et donc plus importantes ont plus d'effets. Ainsi, chez les animaux également ont observé un effet protecteur du Spirulysat^{®} notamment sur le foie, les reins, le coeur ou le système nerveux.

Ainsi, le Spirulysat^{®} donne des effets à partir de 32 mg de Phycocyanine, soit 10 fois moins que ce qui est décrit dans l'art antérieur, c'est-à-dire 350mg-900mg de Phycocyanine, ce qui le rend très abordable d'un point de vue industriel, mais également pour le patient.

De manière avantageuse, une dose de 10 ampoules par jour, c'est-à-dire la prise de Spirulysat^{®} concentré à 100mg de Phycocyanine et 15 mg de polysaccharides par jour, permet une diminution encore plus importante des effets secondaires indésirables, tels que les neuropathies périphériques, induits par un traitement anti-cancéreux, comme les traitements aux sels de platine et notamment les traitements à base d'oxaliplatine.

En particulier, les inventeurs ont montré que la prise de Spirulysat^{®} concentré à 100mg de Phycocyanine et 15 mg de polysaccharides par jour, permet de faire baisser de 45% à 10% le taux de patients sous traitement oncologique aux sels de platine et plus particulièrement sous oxaliplatine, présentant des effets secondaires indésirables au moins très graves comme les neuropathie périphériques chimio-induites (voir tableau 1 ci-dessous). 90% des patients n'ont que des effets mineurs.

**[Tableau 1]**

| Concentration Spirulysat^{®} | 0 mg/j | 100mg/j |
|---|---|---|
| % patients atteints de neuropathies chimio-induites | 45% | 10% |

L'effet du Spirulysat^{®} est donc lié à la dose. Une dose de 50 mg/jour minimum pour un adulte est recommandée. Pour un enfant elle est à adapter à sa masse corporelle. Les inventeurs ont montré qu'une dose de 100 mg/jour permet d'avoir des résultats encore meilleurs, ou un taux de diminution des effets secondaires plus importants sur la population.

Le seuil de tolérance du Spirulysat^{®} d'un point de vue toxicité est au-delà de 1.4 litres de Spirulysat^{®} par jour pour un adulte, selon les dernières études sur modèles animaux. Certains patients réagissent beaucoup plus facilement au Spirulysat^{®} et obtiennent des bons résultats pour 5 ampoules par jour (soit 50mg/jour de Phycocyanine et 7,5 mg/jour de polysaccharides de Spiruline). D'autres ont eu besoin de 10 ampoules par jour (soit 100mg/jour de Phycocyanine et 15 mg/jour de polysaccharides de Spiruline). Une personnalisation et une adaptation de la prise de Spirulysat^{®} est donc possible en fonction des patients, tout en permettant de rester en dessous des dosages décrit dans l'art antérieur.

Ainsi, le Spirulysat^{®}, alliant plusieurs molécules d'intérêt de la Spiruline dont la Phycocyanine, et les polysaccharides de Spiruline, offre l'opportunité d'une personnalisation de concentration pour répondre au plus près aux besoins des patients.

En outre, la prise de Spirulysat^{®} permet aux patients de mieux supporter leur traitement et donc, d'éviter ou limiter l'adaptation posologique du traitement en cours, contrairement à ce qui a pu être observé dans 50% des cas de traitement anti-cancéreux sans prise de Spirulysat^{®}. En effet, les inventeurs ont mis en évidence que la prise de 20mg/jour de Spirulysat^{®} permet de diminuer à 10% les cas nécessitant une adaptation posologique (voir tableau 2 ci-dessous). Avec une prise de 50mg/jours et 100mg/jours plus aucune adaptation posologique n'est nécessaire.

Ainsi, les patients respectent mieux leur protocole de traitement, ce qui contribue à renforcer son efficacité, tout en bénéficiant d'une amélioration de leur bien-être au niveau : alimentation, sommeil, activité physique, résistance au stress.

**[Tableau 2]**

| Concentration Spirulysat^{®} | 0 mg/j | 20mg/j | 50mg/j | 100mg/j |
|---|---|---|---|---|
| % de cas d'adaptation posologique | >50% | 10% | 0% | 0% |

De manière avantageuse, le Spirulysat^{®} n'intervient pas comme un soin médicamenteux mais comme un accompagnement thérapeutique contribuant à rendre les traitements médicaux plus efficaces. Il s'agit d'une innovation majeure en termes de santé : pour la première fois, l'association d'un traitement médical et d'un complément alimentaire naturel aboutit à créer un cocktail puissant et incroyablement efficace au bénéfice des patients.

## Revendications

1. Composition pour utilisation dans le traitement et/ou la prévention des neuropathies périphériques chimio-induites et leurs symptômes induits par un composé anti-cancéreux, ladite composition comprenant un extrait liquide aqueux, ledit extrait liquide aqueux comprenant de la Phycocyanine.

2. Composition pour utilisation selon la revendication 1, **caractérisée en ce que** ledit extrait liquide aqueux comprend en outre des polysaccharides de Spiruline.

3. Composition pour utilisation selon l'une quelconque des revendications 1 à 2, **caractérisée en ce que** ledit composé anti-cancéreux appartient au groupe comprenant un sel de platine, un taxane, une anthracycline.

4. Composition pour utilisation selon la revendication 3, **caractérisée en ce que** ledit composé anti-cancéreux est choisi parmi les sels de platine ; de préférence parmi le groupe comprenant le cisplatine, la carboplatine, l'oxaliplatine et leur mélange.

5. Composition pour utilisation selon l'une quelconque des revendications 1 à 4, **caractérisée en ce que** ledit extrait aqueux liquide comprend entre 20 et 150 mg, préférentiellement entre 50 et 120 mg, très préférentiellement 100 mg de Phycocyanine.

6. Composition pour utilisation selon l'une quelconque des revendications 2 à 5, **caractérisée en ce que** ledit extrait aqueux liquide comprend en outre entre 5 et 25 mg, très préférentiellement 20 mg de polysaccharides de Spiruline.

7. Composition pour utilisation selon l'une quelconque des revendications 1 à 6, **caractérisée en ce que** ladite composition comprend uniquement ledit extrait aqueux liquide.

8. Composition pour utilisation selon la revendication 7, **caractérisée en ce qu'**elle est administrée sous forme liquide par voie orale.

9. Composition pour utilisation selon l'une quelconque des revendications 7 à 8, **caractérisée en ce que** ladite composition est administrée à une dose comprise entre 20 mg/jour et 150 mg/jour, préférentiellement entre 50mg/jour et 150 mg/jours, très préférentiellement à 100 mg/jour.

10. Extrait liquide aqueux pour l'utilisation dans le traitement et/ou la prévention des les neuropathies périphériques chimio-induites et leurs symptômes induits par un composé anti-cancéreux, ledit extrait liquide aqueux comprenant de la Phycocyanine et optionnellement des polysaccharides de Spiruline, obtenu à partir de cyanobactéries.

11. Extrait liquide aqueux pour son utilisation selon la revendication 10, **caractérisée en ce que** ledit composé anti-cancéreux appartient au groupe comprenant un sel de platine, un taxane, une anthracycline.

12. Extrait liquide aqueux pour son utilisation selon la revendication 11, **caractérisée en ce que** ledit composé anti-cancéreux est choisi parmi les sels de platine ; de préférence parmi le groupe comprenant le cisplatine, la carboplatine, l'oxaliplatine et leur mélange.

## Patentansprüche

1. Zusammensetzung zur Verwendung bei der Behandlung und/oder Vorbeugung chemisch induzierter peripherer Neuropathien und ihrer durch eine Antikrebsverbindung induzierten Symptome, wobei die Zusammensetzung einen wässrigen flüssigen Extrakt umfasst, wobei der wässrige flüssige Extrakt Phycocyanin umfasst.

2. Zusammensetzung zur Verwendung nach Anspruch 1, **dadurch gekennzeichnet, dass** der wässrige flüssige Extrakt außerdem Spirulina-Polysaccharide enthält.

3. Zusammensetzung zur Verwendung nach einem der Ansprüche 1 bis 2, **dadurch gekennzeichnet, dass** die Antikrebsverbindung zu der Gruppe gehört, die ein Platinsalz, ein Taxan, ein Anthracyclin umfasst.

4. Zusammensetzung zur Verwendung nach Anspruch 3, **dadurch gekennzeichnet, dass** die Antikrebsverbindung aus Platinsalzen, vorzugsweise aus der Gruppe, die Cisplatin, Carboplatin, Oxaliplatin und deren Gemisch umfasst, ausgewählt ist.

5. Zusammensetzung zur Verwendung nach einem der Ansprüche 1 bis 4, **dadurch gekennzeichnet, dass** der wässrige flüssige Extrakt zwischen 20 und 150 mg, bevorzugt zwischen 50 und 120 mg, besonders bevorzugt 100 mg Phycocyanin enthält.

6. Zusammensetzung zur Verwendung nach einem der Ansprüche 2 bis 5, **dadurch gekennzeichnet, dass** der wässrige flüssige Extrakt außerdem zwischen 5 und 25 mg, besonders bevorzugt 20 mg Spirulina-Polysaccharide enthält.

7. Zusammensetzung zur Verwendung nach einem der Ansprüche 1 bis 6, **dadurch gekennzeichnet, dass** die Zusammensetzung nur den wässrigen flüssigen Extrakt umfasst.

8. Zusammensetzung zur Verwendung nach Anspruch 7, **dadurch gekennzeichnet, dass** sie in flüssiger Form auf oralem Weg verabreicht wird.

9. Zusammensetzung zur Verwendung nach einem der Ansprüche 7 bis 8, **dadurch gekennzeichnet, dass** die Zusammensetzung in einer Dosis zwischen 20 mg/Tag und 150 mg/Tag, bevorzugt zwischen 50 mg/Tag und 150 mg/Tag, besonders bevorzugt zu 100 mg/Tag verabreicht wird.

10. Wässriger flüssiger Extrakt zur Verwendung bei der Behandlung und/oder Vorbeugung chemisch induzierter peripherer Neuropathien und ihrer durch eine Antikrebsverbindung induzierten Symptome, wobei der wässrige flüssige Extrakt Phycocyanin und optional Spirulina-Polysaccharide enthält und aus Cyanobakterien gewonnen wird.

11. Wässriger flüssiger Extrakt zur Verwendung nach Anspruch 10, **dadurch gekennzeichnet, dass** die Antikrebsverbindung zu der Gruppe gehört, die ein Platinsalz, ein Taxan, ein Anthracyclin umfasst.

12. Wässriger flüssiger Extrakt zur Verwendung nach Anspruch 11, **dadurch gekennzeichnet, dass** die Antikrebsverbindung aus Platinsalzen, vorzugsweise aus der Gruppe, die Cisplatin, Carboplatin, Oxaliplatin und deren Gemisch umfasst, ausgewählt ist.

## Claims

1. A composition for use in the treatment and/or prevention of chemo-induced peripheral neuropathies and their symptoms induced by an anti-cancer compound, said composition comprising an aqueous liquid extract, said aqueous liquid extract comprising Phycocyanin.

2. The composition for use according to claim 1, **characterised in that** said aqueous liquid extract further comprises Spirulina polysaccharides.

3. The composition for use according to any one of claims 1 to 2, **characterised in that** said anti-cancer compound belongs to the group comprising a platinum salt, a taxane, an anthracycline.

4. The composition for use according to claim 3, **characterised in that** said anti-cancer compound is selected from platinum salts; preferably from the group comprising cisplatin, carboplatin, oxaliplatin and their mixture.

5. The composition for use according to any one of claims 1 to 4, **characterised in that** said liquid aqueous extract comprises between 20 and 150 mg, preferably between 50 and 120 mg, very preferably 100 mg of Phycocyanin.

6. The composition for use according to any one of claims 2 to 5, **characterised in that** said liquid aqueous extract further comprises between 5 and 25 mg, very preferably 20 mg of Spirulina polysaccharides.

7. The composition for use according to any one of claims 1 to 6, **characterised in that** said composition comprises only said liquid aqueous extract.

8. The composition for use according to claim 7, **characterised in that** it is administered orally in liquid form.

9. The composition for use according to any one of claims 7 to 8, **characterised in that** said composition is administered at a dose comprised between 20 mg/day and 150 mg/day, preferably between 50 mg/day and 150 mg/day, very preferably at 100 mg/day.

10. An aqueous liquid extract for use in the treatment and/or prevention of chemo-induced peripheral neuropathies and their symptoms induced by an anti-cancer compound, said aqueous liquid extract comprising Phycocyanin and optionally Spirulina polysaccharides, obtained from cyanobacteria.

11. The aqueous liquid extract for its use according to claim 10, **characterised in that** said anti-cancer compound belongs to the group comprising a platinum salt, a taxane, an anthracycline.

12. The aqueous liquid extract for its use according to claim 11, **characterised in that** said anti-cancer compound is selected from platinum salts; preferably from the group comprising cisplatin, carboplatin, oxaliplatin and their mixture.
